Europäisches Patentamt

European Patent Office

Office européen des brevets

(19)

(11) Veröffentlichungsnummer : **0 414 115 B1**

(12) **EUROPÄISCHE PATENTSCHRIFT**

(45) Veröffentlichungstag der Patentschrift :
**10.03.93 Patentblatt 93/10**

(51) Int. Cl.⁵ : **A61K 31/525, A61K 9/16**

(21) Anmeldenummer : **90115636.4**

(22) Anmeldetag : **16.08.90**

(54) **Hilfsmittelfreie Riboflavingranulate.**

(30) Priorität : **23.08.89 DE 3927810**

(43) Veröffentlichungstag der Anmeldung :
**27.02.91 Patentblatt 91/09**

(45) Bekanntmachung des Hinweises auf die
Patenterteilung :
**10.03.93 Patentblatt 93/10**

(84) Benannte Vertragsstaaten :
**AT BE CH DE DK FR GB IT LI NL SE**

(56) Entgegenhaltungen :
**EP-A- 0 219 276**
**EP-A- 0 307 767**
**EP-A- 0 345 717**

(56) Entgegenhaltungen :
**P.H. LIST : "Arnzeiformenlehre", Auflage 1,
1976, Seiten 19-20, Wissenschaftliche Verlagsgesellschaft, Stuttgart, DE**
**R. VOIGT : "Lehrbuch der pharmazeutischen
Technologie", 1976, Seite 613, Verlag Chemie,
Berlin, DE**

(73) Patentinhaber : **BASF Aktiengesellschaft
Carl-Bosch-Strasse 38
W-6700 Ludwigshafen (DE)**

(72) Erfinder : **Buehler, Volker, Dr.
In den Weingaerten 14
W-6706 Wachenheim (DE)**
Erfinder : **Petersen, Hermann, Dr.
Colgensteiner Weg 14
W-6718 Gruenstadt (DE)**

**Beschreibung**

Die Erfindung betrifft ein hilfsmittelfreies Riboflavingranulat, dessen mittlerer größter Teilchendurchmesser 50 - 1000 μm beträgt, das dadurch gekennzeichnet ist, daß es aus Riboflavinpulver, dessen mittlerer größter Teilchendurchmesser kleiner als 25 μm betrug, dadurch erhalten wurde, daß das Pulver nach dem Kompaktierverfahren zu Strängen oder Bändern verpreßt und diese in an sich bekannter Weise auf die entsprechende Teilchengröße zerkleinert wurden, ein Verfahren zu seiner Herstellung sowie dessen Verwendung zur Bereitung von Riboflavin enthaltenden Arznei-, Lebens- und Futtermitteln, insbesondere von Tabletten.

Riboflavin (Vitamin B2) fällt bei seiner synthetischen Herstellung oder der biotechnischen Gewinnung in Form von sehr feinkristallinen Pulvern an, deren Teilchen einen mittleren größten Durchmesser von etwa 20 μm haben.

Da ein solches Pulver stark staubt, eine sehr geringe Schüttdichte hat - meist unter 0,2 g/ml -, sich elektrostatisch auflädt und nur sehr schlecht fließt, läßt es sich nur mit großen Problemen weiterverarbeiten. Vor allem ist es nicht zur Herstellung von Tabletten geeignet, deren Riboflavinanteil 25 Gew.% übersteigt (V. Bühler, "Vademecum for Vitamin Formulations", Wissenschaftliche Verlagsgesellschaft, Stuttgart, Seite 98 - 99).

Man bringt das Riboflavin deshalb in Form von Granulaten in den Handel, die mit Hilfe eines Bindemittels hergestellt werden (s. z.B. EP-A-0 219 276).

Obwohl sich diese Granulate verfahrenstechnisch gut für die Weiterverarbeitung eignen, sei es zur Direkttablettierung, zur Bereitung anderer Riboflavin enthaltender Arzneimittelpräparate oder von Vitamin B2 enthaltenden Lebens- und Futtermitteln, bleibt es häufig unbefriedigend, daß sie nicht aus reinem Wirkstoff bestehen.

Der Erfindung lag daher die Herstellung eines Granulats aus hilfsmittelfreiem Riboflavin als Aufgabe zugrunde.

Als Lösung dieser Aufgabe wurde ein Verfahren zur Herstellung des oben beschriebenen Riboflavingranulats gefunden, welches dadurch gekennzeichnet ist, daß man hilfsmittelfreies Riboflavinpulver, dessen mittlerer größter Teilchendurchmesser kleiner ist als 25 μm, nach dem Kompaktierverfahren zu Strängen oder Bändern verpreßt und diese in an sich bekannter Weise auf die entsprechende Teilchengröße zerkleinert.

Das Kompaktierverfahren ist an sich bekannt. Es wird bei einer großen Anzahl technischer Produkte wie Chromdioxid, Düngemitteln, Pflanzenschutzmitteln und in geringerem Umfang in der pharmazeutischen Industrie eingesetzt. Es besteht darin, daß man ein Produktpulver zwischen zwei gegenläufig rotierenden Walzen zu Strängen oder Bändern verpreßt. Die Walzen können völlig glatt sein oder Vertiefungen definierter Form haben. Außerdem besteht die Möglichkeit, beheizbare oder kühlbare Walzen einzusetzen. Nach dem Durchgang des (nunmehr kompaktierten) Gutes kann dieses mit Hilfe von Siebzerkleinerern, Stachel- oder Riffelwalzen gebrochen und gegebenenfalls nach üblichen Verfahren schonend weiter zerkleinert und durch anschließendes Sieben fraktioniert werden. Staubanteile und zu große Teilchen können dem Prozeß wieder zugeführt werden [s. z.B. R. Voigt, "Lehrbuch der pharmazeutischen Technologie, Verlag Chemie, (1975), S. 163; K.H. Bauer, K.H. Frömming und C. Führer, "Pharmazeutische Technologie", Georg Thieme Verlag Stuttgart, New York, (1986) S. 367 - 368]

Daß sich dieses Verfahren im vorliegenden Fall mit Erfolg anwenden läßt, war im Hinblick darauf, daß sich das Riboflavinpulver nicht zu stabilen Tabletten verpressen läßt, nicht zu erwarten.

Preßkräfte von 5 bis 20 kN/cm Walzenbreite, vorzugsweise von 7 bis 13 kN/cm sind bei der Kompaktierung von Riboflavin besonders empfehlenswert. Das Verpressen erfolgt in der Regel bei 0 bis 50°C, vorzugsweise bei etwa 25°C.

Das erfindungsgemäße Riboflavingranulat, dessen Staubanteil (größter Teilchendurchmesser unter 50 μm) unter 15 % liegt, ist problemlos weiterverarbeitbar. Besondere Vorteile bietet es bei der (Direkt-)Tablettierung mit den hierfür üblichen Tablettierungshilfsstoffen für sich allein oder zusammen mit anderen Wirkstoffen. Auch verfahrenstechnisch erfolgt die Tablettierung in bekannter Weise mittels Direkttablettierung oder nach Feuchtgranulation, so daß sich nähere Angaben hierzu erübrigen.

Die so erhältlichen Tabletten haben auch bei hohen Riboflavingehalten von über 50 Gew.% so gute mechanische Eigenschaften wie diejenigen, die mit bindemittelhaltigen Granulaten erhältlich sind.

Das Granulat eignet sich hervorragend zur Herstellung von Riboflavin enthaltenden Arznei-, Lebens- und Futtermitteln. Im Falle der Futtermittel braucht es nicht hochrein zu sein, sondern kann noch Biomasse enthalten, die von der fermentativen Herstellung des Riboflavins stammt. Im Falle von Lebensmitteln stellt die Mehlvitaminierung ein wichtiges Einsatzgebiet dar.

Beispeile A, B, C

Riboflavinpulver einer mittleren Teilchengröße von 20 μm wurde in drei Einzelversuchen A, B und C an einem Walzen-Kompaktier-Apparat zu Bändern verpreßt; die Preßkraft betrug zwischen 7 - 10 kN/cm Walzenbreite. Anschließend wurden die so erhaltenen Bänder mit Hilfe eines Siebzerkleinerers zerkleinert (Maschenweite 630 μm).

Die so erhaltenen Granulate hatten mittlere größte Teilchendurchmesser von unter 600 μm.

Einzelheiten zu den Versuchen sowie über die anwendungstechnischen Eigenschaften des Granulats und daraus hergestellter Tabletten sind den nachfolgenden Tabellen zu entnehmen.

| | Riboflavin-pulver | Riboflavingranulate | | | |
|---|---|---|---|---|---|
| | | A | B[1] | C | Vergleich[2] |
| Preßkraft [kN/cm] | – | 8 | 7 | 10 | – |
| Teilchengröße [μm] | 20 | < 630 | < 450 | < 630 | – |
| Schüttdichte [g/cm$^{-3}$] | < 0.2 | 0.53 | 0.44 | 0.51 | 0.56 |
| Staubtest[3] nach 0 sek | | 4 | 11 | 4 | 17 |
| 10 | | 3 | 7 | 3 | 12 |
| 30 | | 2 | 4 | 2 | 8 |
| Fließverhalten | sehr schlecht | gut | befriedigend bis gut | gut | gut |
| Elektrostatische Aufladung | stark | sehr schwach | sehr schwach | sehr schwach | sehr schwach |

Verhalten in einer Tablettenformulierung[4]

|  | Riboflavin-pulver | Riboflavingranulate | | | Vergleich[2] |
|---|---|---|---|---|---|
|  |  | A | B[1] | C |  |
| Preßverhalten | nicht verpreßbar | gut | gut | gut | gut |
| Härte [N] [5] | - | 109 | 95 | 116 | 105 |
| Zerfall [min] [6] | - | 2 | 3 | 3 | 7 |
| Abrieb [%] [7] | - | 0.1 | 0.1 | 0.1 | 0.2 |

[1]) Der Grobanteil über 500 $\mu$m wurde abgesiebt.

[2]) Das kommerzielle Vergleichsgranulat enthielt 95 Gew.-% Riboflavin, ca. 4 Gew.-% Gummi arabicum und etwas Silicagel

[3]) Staubtest nach Casella. Gemessen wird die Reduktion der Lichtdurchlässigkeit in einer Cassella-Staubkammer. Je höher die Reduktion der Lichtdurchlässigkeit ist, desto schlechter ist das Staubverhalten einer Substanz zu beurteilen.

[4]) Die Direkttablettierungsformulierung bestand pro Tablette aus:

156 mg Riboflavingranulat
140 mg Lactose
  5 mg Polyvinylpyrrolidon K-Wert* 30
  5 mg Unlösliches Polyvinylpolypyrrolidon
  4 mg Magnesiumstearat
  2 mg Kieselsäure

[5]) Härtetester von Heberlein/Schleuninger

[6]) Prüfung in künstlichem Magensaft nach der Europäischen Pharmakopöe, Kapitel V. 5.1.1.

[7]) Gemessen im Roche-Friabilator

* US-Pharmakopeia 22, Monographie "Povidone", S. 1118

Wie die Tabelle zeigt, erwiesen sich im Vergleich zu Riboflavin-Pulver die erfindungsgemäßen hilfsstofffreien Riboflavin-Granulate sowohl in ihren verarbeitungstechnischen Eigenschaften als auch in Tabletten als

4

weit überlegen. Im Vergleich zu einem kommerziellen hilfsstofffhaltigen Riboflavin-Granulat zeigten die erfindungsgemäßen hilfsstofffreien Granulate ein besseres Verhalten im Staubtest und beim Tablettenzerfall und zumindest etwa gleich gute andere Eigenschaften.

Beispiel D

Analog zu Beispiel C wurde Riboflavinpulver mit einer mittleren Teilchengröße von 20 μm einem Walzen-Kompaktier-Apparat mit einer Preßkraft von 10 kN/cm Walzenbreite verpreßt. Anschließend wurden die erhaltenen Bänder mit Hilfe eines Siebzerkleinerers zerkleinert (Maschenweite 630 μm).

Das Produkt wurde in niederdosierten Riboflavintabletten zur Prüfung der Gleichförmigkeit des Gehaltes nach der Europäischen Pharmakopöe (Kapitel V. 5.2.2.) in folgender Formulierung verarbeitet:

3 mg Riboflavin-Granulat
7 mg Polyvinylpyrrolidon vom K-Wert 30
7 mg unlösliches Polyvinylpyrrolidon
181 mg Lactose
2 mg Magnesiumstearat
1 mg Kieselsäure

500 g dieser Mischung wurden durch ein 1-mm-Sieb gegeben, 20 min. im Kubusmischer gemischt und mit sehr niedrigem Preßdruck (4 kN) auf einem Rundläufer zu Tabletten verpreßt.

Die allgemeinen Tabletteneigenschaften im Vergleich zu Tabletten, die mit kommerziellen hilfsstoffhaltigen Riboflavingranulaten hergestellt wurden, sind der folgenden Tabelle zu entnehmen.

| | | Beispiel D | Vergleich[2] | Vergleich[8] |
|---|---|---|---|---|
| Gewicht | [mg] | 208 | 202 | 205 |
| Durchmesser | [mm] | 8 | 8 | 8 |
| Härte | [N] [5] | 97 | 108 | 100 |
| Zerfall | [min] [6] | 3-4 | 4 | 5 |
| Abrieb | [%] [7] | 0,1 | 0,1 | 0,1 |
| Riboflavin | [%] | 1,43 | 1,40 | 1,38 |

[2] Das kommerzielle Vergleichsgranulat enthielt 95 Gew.-% Riboflavin, ca. 4 Gew.-% Gummi arabicum und etwas Silicagel

[5] Härtetester von Heberlein/Schleuninger

[6] Prüfung in künstlichem Magensaft nach der Europäischen Pharmakopöe, Kapitel V. 5.1.1.

[7] Gemessen im Roche-Friabilator

[8] Das kommerzielle Vergleichsgranulat enthielt 98 Gew.-% Riboflavin und ca. 2 % Methylcellulose als Hilfsstoff.

Die Gleichförmigkeit des Riboflavin-Gehaltes von 20 Tabletten geht aus der folgenden Tabelle hervor:

| Tabletten-Nr. | Bsp. D [%] | Vergleich[2] [%] | Vergleich[8] [%] |
|---|---|---|---|
| 1 | 1,482 | 1,307 | 1,822 |
| 2 | 1,462 | 1,383 | ./. |
| 3 | 1,521 | 1,273 | 1,172 |
| 4 | 1,413 | 1,308 | 1,728* |
| 5 | 1,252 | 1,415 | 1,546 |
| 6 | 1,349 | 1,440 | 0,990* |
| 7 | 1,340 | 1,234 | 1,133 |
| 8 | 1,379 | 1,796* | 1,450 |
| 9 | 1,354 | 1,573 | 0,832* |
| 10 | 1,584 | 1,314 | 2,040* |
| 11 | 1,446 | 1,263 | 1,089 |
| 12 | 1,539 | 1,353 | 1,634 |
| 13 | 1,483 | 1,554 | 0,807* |
| 14 | 1,449 | 1,186 | 1,656 |
| 15 | 1,380 | 1,365 | 1,639 |
| 16 | 1,536 | 1,555 | 1,054 |
| 17 | 1,265 | 1,523 | 1,110 |
| 18 | 1,447 | 1,343 | 1,792* |
| 19 | 1,537 | 1,610 | 1,665 |
| 20 | 1,352 | 1,264 | 1,030* |
| Durchschnitt | 1,4285 | 1,4030 | 1,3784 |
| rel. Standardabweichung | 0,093 | 0,155 | 0,374 |
| +/- 25 % des Durch-schnitts | 1,0714-1,7856 | 1,0522-1,7537 | 1,0338-1,7232 |

*) = außerhalb der vorgeschriebenen Grenzen

Bei dem erfindungsgemäßen hilfsstofffreien Riboflavin-Granulat betrugen die maximalen Abweichungen + 8 % bzw. - 12 % vom Durchschnitt. Bei den kommerziellen hilfsstoffhaltigen Riboflavin-Granulaten waren die Abweichungen wesentlich größer und überstiegen teilweise sogar die vorgeschriebenen Maximalgrenzen von +/- 25 % (durch * gekennzeichnet).

Beispiel E

Riboflavinpulver mit einer mittleren Teilchengröße von 20 µm wurde in einem Walzen-Kompaktier-Apparat mit einer Preßkraft von etwa 0,9 kN/cm Walzenbreite verpreßt. Anschließend wurden die so erhaltenen Bänder mit Hilfe zweier nacheinandergeschalteter Siebzerkleinerer (Maschenweiten 630 µm und 315 µm) zerkleinert.

Zur Prüfung der Verwendbarkeit in einem praxisüblichen Prämix zur Mehlvitaminierung wurde das so erhaltene Riboflavin-Granulat in folgende Mischung eingearbeitet (5-l-Gefäß mit Pflugscharmischer, Drehzahl 150 Upm, Mischzeit 30 min):

3 % Riboflavin-Granulat

4 % Thiaminmononitrat

30 % Nicotinsäureamid

2 % Tricalciumphosphat

25 % reduziertes Eisenpulver

36 % Maisstärke

Wie am Markt üblich, wurde diese Mischung durch ein 200 μm-Rüttelsieb gegeben, um grobere Partikeln und/oder Agglomerate (auch von Riboflavin) zu eliminieren und eine homogene Verteilung im Mehl zu erzielen. Ein wichtiges Ziel muß es deshalb sein, diesen Rückstand so klein wie möglich zu halten.

Die Rückstandsmengen einer Versuchsreihe sind im Vergleich zu feinkristallinem Riboflavinpulver in der folgenden Tabelle aufgeführt:

Anteil an Riboflavin (%) in 200 μm-Siebrücklsand, bezogen auf eingesetztes Riboflavin

|                        | Beispiel E | Riboflavinpulver |
|------------------------|------------|------------------|
|                        | 3,88       | 7,70             |
|                        | 4,07       | 2,20             |
|                        | 2,76       | 1,93             |
|                        | 3,53       | 2,92             |
|                        | 3,79       | 4,18             |
|                        | 4,13       |                  |
| Durchschnitt           | 3,69       | 3,79             |
| rel. Standardabweichung| 0,461      | 2,11             |

Wie die Ergebnisse zeigen, ist das erfindungsgemäße, hilfsstofffreie Riboflavin-Granulat - bei signifikant verbesserten Produkteigenschaften wie gutem Fließverhalten, geringer Staubneigung und geringer elektrostatischer Aufladung (siehe Beispiele A-C) - mindestens im gleichen Maße für die Mehlvitaminierung geeignet wie feinkristallines Riboflavinpulver.

**Patentansprüche**

1. Hilfsmittelfreies Riboflavingranulat, dessen mittlerer größter Teilchendurchmesser 50 bis 1000 μm be-trägt, das dadurch gekennzeichnet ist, daß es aus Riboflavinpulver, dessen mittlerer größter Teilchen-durchmesser kleiner als 25 μm betrug, dadurch erhalten wurde, daß das Pulver nach dem Kompaktierverfahren zu Strängen oder Bändern verpreßt und diese in an sich bekannter Weise auf die entsprechende Teilchengröße zerkleinert wurden.

2. Riboflavingranulat nach Anspruch 1, dessen mittlerer größter Teilchendurchmesser 100 bis 1000 μm be-trägt.

3. Verfahren zur Herstellung eines Riboflavingranulates gemäß Anspruch 1 oder 2, dadurch gekennzeichnet, daß man Riboflavinpulver, dessen mittlerer größer Teilchendurchmesser kleiner als 25 μm ist, nach dem Kompaktierverfahren zu Strängen oder Bändern verpreßt und diese in an sich bekannter Weise auf die entsprechende Teilchengröße zerkleinert.

4. Verwendung eines Riboflavingranulates gemäß Anspruch 1 oder 2 zur Bereitung von Arznei-, Lebens, und Futtermitteln.

5. Verwendung eines Riboflavingranulates gemäß Anspruch 1 oder 2 zur Herstellung von Tabletten.

6. Aus Riboflavingranulat gemäß Anspruch 1 hergestellte Tabletten.

**Claims**

1. Riboflavin granules containing no inactive ingredient and having a mean maximum particle diameter of from 50 to 1,000 μm, which have been obtained from riboflavin powder whose mean maximum particle diameter is below 25 μm by compacting the powder to give strands or ribbons, and reducing the latter to the appropriate particle size in a conventional manner.

2. Riboflavin granules as claimed in claim 1, having a mean maximum particle diameter of from 100 to 1,000 µm.

3. A process for producing riboflavin granules as claimed in claim 1 or 2, which comprises compacting riboflavin powder whose mean maximum particle diameter is below 25 µm to give strands or ribbons, and reducing the latter to the appropriate particle size in a conventional manner.

4. Use of riboflavin granules as claimed in claim 1 or 2 for preparing drugs and human and animal foods.

5. Use of riboflavin granules as claimed in claim 1 or 2 for producing tablets.

6. Tablets produced from riboflavin granules as claimed in claim 1.

## Revendications

1. Granulé de riboflavine sans excipient, dont le diantre de particules moyen le plus grand est de 50 à 1000 µm, qui est caractérisé en ce qu'il est obtenu à partir de poudre de riboflavine dont le diamètre de particules moyen le plus grand est plus petit que 25 µm, en ce que la poudre est comprimée sous presse selon un procédé de compactage, en barres ou rubans et en ce que ceux-ci sont broyés d'une manière connue en soi pour atteindre la taille de particules désirée.

2. Granulé de riboflavine selon la revendication 1, dont le diantre de particules moyen le plus grand est de 100 à 1000 µm.

3. Procédé de préparation d'un granulé de riboflavine selon la revendication 1 ou 2, caractérisé en ce que l'on comprime sous presse une poudre de riboflavine dont le diamètre de particules moyen le plus grand est plus petit que 25 µm, selon un procédé de compactage, pour obtenir des barres ou des rubans, et en ce que l'on broie ceux-ci d'une manière connue en soi jusqu'à l'obtention de la taille de particules désirée.

4. Utilisation d'un granulé de riboflavine selon la revendication 1 ou 2, pour la préparation de médicaments, d'aliments pour l'homme et l'animal.

5. Utilisation d'un granulé de riboflavine selon la revendication 1 ou 2, pour la préparation de comprimés.

6. Comprimés préparés à partir de granulés de riboflavine selon la revendication 1.